# EUROPEAN PATENT SPECIFICATION

(11) **EP 3 584 866 B1**
(45) Date of publication and mention of the grant of the patent: **13.12.2023**
(21) Application number: 18753801.2
(22) Date of filing: 13.02.2018
(51) Int. Cl.: H01M 8/18, H01M 8/08

(54) **ELECTROLYTIC SOLUTION, ELECTROLYTIC AQUEOUS SOLUTION, AND POWER GENERATING DEVICES**
ELEKTROLYTLÖSUNG, WÄSSRIGE ELEKTROLYTLÖSUNG, UND STROMERZEUGUNGSVORRICHTUNGEN
SOLUTION ÉLECTROLYTIQUE, SOLUTION AQUEUSE ÉLECTROLYTIQUE, ET DISPOSITIFS DE GÉNÉRATION D'ÉNERGIE

(30) Priority: 17.02.2017 JP 2017028443; 17.02.2017 JP 2017028444
(43) Date of publication of application: 25.12.2019
(73) Proprietor: Kyushu University, National University Corporation, Nishi-ku, Fukuoka-shi Fukuoka 819-0395 (JP)
(72) Inventor: HOSHINO Yu, Fukuoka-shi Fukuoka 819-0395 (JP); GUO Benshuai, Fukuoka-shi Fukuoka 819-0395 (JP); YAMADA Teppei, Fukuoka-shi Fukuoka 819-0395 (JP); GAO, Fan., Fukuoka-shi Fukuoka 819-0395 (JP); UEDA Kentaro, Osaka-shi Osaka 541-0046 (JP)
(74) Representative: LBP Lemcke, Brommer & Partner Patentanwälte mbB
(86) International application number: PCT/JP2018/004891
(87) International publication number: WO 2018/151098

(56) References cited:
- WO-A1-2013/027668
- WO-A1-2015/148357
- WO-A1-2016/144909
- JP-A- H04 190 572
- JP-A- 2002 100 398
- JP-A- 2002 305 841
- JP-A- 2015 534 708
- JP-A- 2016 533 613
- US-A- 3 294 588
- J. BRIJITTA ET AL: "Phase Behavior of Poly(N-isopropylacrylamide) Nanogel Dispersions: Temperature Dependent Particle Size and Interactions", JOURNAL OF NANOSCIENCE AND NANOTECHNOLOGY, vol. 9, no. 9, 1 September 2009 (2009-09-01), pages 5323-5328, XP055197999, ISSN: 1533-4880, DOI: 10.1166/jnn.2009.1144

## Description

### Technical Field

The present invention relates to an electrolytic solution, an electrolytic aqueous solution, and a power generating device in which the electrolytic solution or the electrolytic aqueous solution is used.

### Background Art

As a technique for converting a temperature gradient to electric energy, thermoelectric conversion elements are known, and thermoelectric conversion elements such as bismuth telluride thermoelectric conversion elements are widely used. However, because they contain expensive and highly toxic elements, their applicable range is limited. Also, the voltage (Seebeck coefficient) generated per temperature difference of 1 °C is as small as about 0.2 mV/K, and thus it has been difficult to bring them into practical use.

Also, systems are known in which a temperature gradient is converted to an ion concentration gradient, and used for conversion to reusable energy or recovering of acid gas. Patent Document 1 discloses a system in which a temperature responsive electrolyte is used, the temperature responsive electrolyte being a substance whose pKa varies significantly according to the temperature. With the temperature responsive electrolyte, an ion concentration gradient is generated, and power generation and the like are performed.
WO 2016/144909 A1 describes redox flow batteries having a first aqueous electrolyte including a first type of redox active material with polycyclic organic compounds such as quinone or alloxazine type compounds, and a second aqueous electrolyte including a second type of redox active material. WO 2015/148357 A1 describes a redox flow battery with an electrolyte the organic redox active molecule of which contains a quinone ring, or a benzoquinone ring system, or an anthraquinone ring system.
JP 2002 100398 A describes a secondary battery in which a quinone compound is dissolved in an aqueous electrolytic solution.
The article "Phase Behavior of Poly(N-isopropylacrylamide) Nanogel Dispersions: Temperature Dependent Particle Size and Interactions" by J. BRIJITTA ET AL, JOURNAL OF NANOSCIENCE AND NANOTECHNOLOGY, vol. 9, no. 9 (Sept. 01, 2009), describes the physical properties of poly(N-isopropylacrylamide) nanoparticles dispersed in aqueous medium as a function of temperature.
JP 2002 305841 A describes a surplus power control system for a power generating company that uses a temperature difference battery unit, which converts heat generated at the power generating facilities into electric energy for storage.
JP H04 190572 A describes a temperature difference battery comprising an electrolyte solution including a redox pair having a redox potential that varies with temperature between a high temperature side electrode and a low temperature side electrode disposed in different temperature ranges.
JP 2016 533613 A and JP 2015 534708 A describe rechargeable flow batteries. Electrical energy is chemically stored in electrochemical electrodes by protonation of small organic molecules called quinones to hydroquinone. US 3 294 588 A describes a fuel cell that has an anolyte compartment with an aqueous anolyte and a cathode compartment with an aqueous catholyte. The catholyte contains a soluble organic compound from the group consisting of quinones, quinone-imines, quinone-diamines, indophenol dyes, indamine dyes, oxazine dyes, thiazine dyes and indigoid dyes.

### Prior Art Document

### Patent Document

Patent Document 1: WO 2013/027668

### Disclosure of the Invention

### Problem to be Solved by the Invention

Patent Document 1 mentions the possibility of application of the system of Patent Document 1 to a temperature difference battery or a fuel cell. The document also mentions a method for generating an ion concentration gradient for achieving a highly efficient fuel cell in which hydrogen and oxygen are used as fuel. However, there is no disclosure as to a specific method for implementing a temperature difference battery.

Also, in the system of Patent Document 1, power generation is performed by utilizing the following electrochemical reaction.

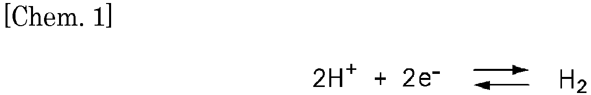

Then, combustible hydrogen gas is generated during extraction of electricity, and it is therefore necessary to provide a structure, function or the like to prevent the hydrogen gas from catching fire in the power generating device. Also, during continuous power generation, the dissolution of generated hydrogen gas in a reaction solution becomes rate-limiting, and thus it is also difficult to increase the reaction speed and improve the power generation efficiency.

The present invention has been made in view of the problems described above, and it is a first object of the present invention to provide an electrolyte that can generate a practically high electromotive force and a high Seebeck coefficient, and a power generating device obtained by using the electrolyte. A second object of the present invention is to provide a method for suppressing generation of hydrogen gas at the time when an ion (hydrogen ion) concentration gradient is generated by a temperature responsive electrolyte.

### Means for Solving Problem

A characteristic configuration of an electrolytic solution for achieving the first object is that the electrolytic solution contains: a temperature responsive electrolyte that is an electrolyte whose pKa varies according to temperature; and an oxidation-reduction active species (excluding a hydroquinone derivative). As the oxidation-reduction active species, it is preferable to use an oxidation-reduction active species whose oxidation-reduction potential varies according to pH. Examples thereof include N,N,N',N'-tetramethyl-p-phenylenediamine, nicotinamide, N-substituted nicotinamide, a proflavine hemisulfate hydrate, riboflavin, anthraquinone, sulfated anthraquinone, methylene blue, dithiothreitol, a ferrocyanide compound, N1-ferrocenylmethyl-N1,N1,N2,N2,N2-pentamethylpropane- 1,2-diaminium dibromide, methylviologen, naphthoquinone, and menadione. Alternatively, derivatives of these compounds or compounds that have structures similar to these compounds may be used. Alternatively, compounds that are used as oxidation-reduction active species in redox flow batteries and the like may be used. It is preferable that a proton conjugated electron transfer reaction is performed. Also, it is desirable that an oxidized form and a reduced form of the oxidation-reduction active species exist together. Here, anthraquinone, sulfated anthraquinone, naphthoquinone, and derivatives thereof do not correspond to hydroquinone derivatives, and they can be used preferably as the oxidation-reduction active species according to the present invention. The term "hydroquinone derivative" used in the specification of the present application refers to a monocyclic aromatic compound that has a hydroquinone skeleton.

The inventors of the present invention conducted in-depth studies and found that the voltage generated per temperature difference of 1°C can be improved by using a temperature responsive electrolyte and an oxidation-reduction active species together. Then, they conducted experiments and confirmed that power generation can be performed by generating a potential difference by using the oxidation-reduction active species and the temperature responsive electrolyte described above, and causing an oxidation-reduction reaction on the electrode surface. In this way, the present invention has been accomplished.

That is, according to the characteristic configuration described above, an oxidation-reduction equilibrium reaction of the oxidation-reduction species involves generation or consumption of protons, and thus with the inclusion of a temperature responsive electrolyte that is an electrolyte whose pKa varies according to temperature, protons generated by the temperature responsive electrolyte can shift the oxidation-reduction equilibrium of oxidation-reduction active species in a direction in which the protons are consumed based on the Le Chatelier's principle. Also, the fact that the oxidation-reduction active species described above can be uniformly dispersed in the temperature responsive electrolyte before and after the oxidation-reduction reaction is also advantageous in terms of suppressing generation of unnecessary precipitates and increasing the reaction speed and the continuity of the reaction. Even when the electrolytic solution is produced by using one of the oxidized form and the reduced form of the oxidation-reduction active species, the other one of the oxidized form and the reduced form of the oxidation-reduction active species is generated in the solution, and an equilibrium state of the oxidation-reduction reaction thereby occurs. Accordingly, such a configuration is also an implementation of the present invention.

According to the present invention, the temperature responsive electrolyte is a molecule that has a polar group, a hydrophobic group, and an ionizable functional group. The temperature responsive electrolyte may also contain a substance that exhibits a phase transition.

A characteristic configuration of a power generating device for achieving the first object is that the power generating device is a power generating device that performs power generation by using the electrolytic solution described above, the power generating device including: a positive electrode; a negative electrode; a heating mechanism; and a cooling mechanism, wherein the positive electrode and the negative electrode are immersed in the electrolytic solution, the heating mechanism heats the electrolytic solution that is present in the vicinity of one of the positive electrode and the negative electrode, and the cooling mechanism cools the electrolytic solution that is present in the vicinity of the other one of the positive electrode and the negative electrode.

According to the characteristic configuration described above, the heating mechanism heats the electrolytic solution that is present in the vicinity of one of the positive electrode and the negative electrode, and the cooling mechanism cools the electrolytic solution that is present in the vicinity of the other one of the positive electrode and the negative electrode. Accordingly, a proton concentration gradient is generated between the vicinity of the positive electrode and the vicinity of the negative electrode, and a potential difference is generated by shifting an oxidation-reduction equilibrium of the oxidation-reduction species. As a result of an oxidation-reduction reaction taking place at this time, power generation can be performed.

As a result of the heating mechanism heating the electrolytic solution to a temperature higher than the phase transition temperature of the substance that is contained in the temperature responsive electrolyte and exhibits a phase transition and the cooling mechanism cooling the electrolytic solution to a temperature lower than the phase transition temperature of the substance that is contained in the temperature responsive electrolyte and exhibits a phase transition, an even larger proton concentration gradient is generated, and the power generation performance of the power generating device can be improved. Accordingly, this configuration is preferable.

The power generating device may be configured as described below. Specifically, the power generating device includes: a positive electrode tank; a negative electrode tank; and a circulation mechanism, the electrolytic solution is contained in the positive electrode tank and the negative electrode tank, the positive electrode is in contact with the electrolytic solution contained in the positive electrode tank, the negative electrode is in contact with the electrolytic solution contained in the negative electrode tank, the heating mechanism heats either one of the electrolytic solution contained in the positive electrode tank and the electrolytic solution contained in the negative electrode tank, the cooling mechanism cools the other one of the electrolytic solution contained in the positive electrode tank and the electrolytic solution contained in the negative electrode tank, and the circulation mechanism circulates the electrolytic solution between the positive electrode tank and the negative electrode tank. With this configuration, a proton concentration gradient is generated between the positive electrode tank and the negative electrode tank by the heating mechanism and the cooling mechanism, and thus electricity can be extracted from the positive electrode and the negative electrode by shifting an oxidation-reduction equilibrium of the oxidation-reduction species. Also, with circulation of the electrolytic solution by the circulation mechanism, the oxidation-reduction equilibrium of the oxidation-reduction species contained in a newly supplied electrolytic solution is constantly shifted, and thus the oxidation-reduction reaction continues, as a result of which power generation can be performed continuously.

Another characteristic configuration of a power generating device according to the present invention is that the power generating device includes a heat exchange mechanism, and the heat exchange mechanism performs heat exchange between the electrolytic solution delivered to the positive electrode tank by the circulation mechanism and the electrolytic solution delivered to the negative electrode tank by the circulation mechanism.

According to the characteristic configuration described above, the energy efficiency of the power generating device can be increased by effectively utilizing heat from the heating mechanism, and thus the characteristic configuration described above is preferable.

A characteristic configuration of an electrolytic aqueous solution for achieving the second object is that the electrolytic aqueous solution contains: a temperature responsive electrolyte that is an electrolyte whose pKa varies according to temperature; and an oxidation-reduction reactive species that is a hydroquinone derivative.

The inventors of the present invention conducted in-depth studies and found that the generation of hydrogen gas can be suppressed by using a temperature responsive electrolyte and an oxidation-reduction reactive species together. Then, they conducted experiments and confirmed that power generation can be performed by using a hydroquinone derivative as the oxidation-reduction reactive species. In this way, the present invention has been accomplished.

That is, according to the characteristic configuration described above, a temperature responsive electrolyte that is an electrolyte whose pKa varies according to temperature and an oxidation-reduction reactive species that is a hydroquinone derivative are contained. Accordingly, protons generated by the temperature responsive electrolyte are consumed by the oxidation-reduction reactive species, and it is therefore possible to suppress generation of hydrogen gas. Also, the fact that the hydroquinone derivative is water soluble before and after the oxidation-reduction reaction, or does not precipitate in the electrolytic aqueous solution is also advantageous in terms of suppressing generation of unnecessary precipitates and increasing the reaction speed and the continuity of the reaction.

According to the present invention, the temperature responsive electrolyte is a molecule that has a polar group, a hydrophobic group, and an ionizable functional group. Also, it has been confirmed through experiments that it is possible to perform power generation by using an electrolytic aqueous solution in which hydroquinone or methyl hydroquinone is used as the oxidation-reduction reactive species.

A characteristic configuration of a power generating device for achieving the second object is that the power generating device is a power generating device that performs power generation by using the electrolytic aqueous solution described above, the power generating device including: a positive electrode; a negative electrode; a heating mechanism; and a cooling mechanism, wherein the positive electrode and the negative electrode are immersed in the electrolytic aqueous solution, the heating mechanism heats the electrolytic aqueous solution that is present in the vicinity of one of the positive electrode and the negative electrode, and the cooling mechanism cools the electrolytic aqueous solution that is present in the vicinity of the other one of the positive electrode and the negative electrode.

According to the characteristic configuration described above, the heating mechanism heats the electrolytic aqueous solution that is present in the vicinity of one of the positive electrode and the negative electrode, and the cooling mechanism cools the electrolytic aqueous solution that is present in the vicinity of the other one of the positive electrode and the negative electrode. Accordingly, an ion concentration gradient is generated between the vicinity of the positive electrode and the vicinity of the negative electrode, and power generation can be performed in a state in which the generation of hydrogen gas is suppressed.

As a result of the heating mechanism heating the electrolytic aqueous solution to a temperature higher than the phase transition temperature of the temperature responsive electrolyte and the cooling mechanism cooling the electrolytic aqueous solution to a temperature lower than the phase transition temperature of the temperature responsive electrolyte, an even larger ion concentration gradient is generated, and the power generation performance of the power generating device can be improved. Accordingly, this configuration is preferable.

The power generating device may be configured as described below. Specifically, the power generating device includes: a positive electrode tank; a negative electrode tank; and a circulation mechanism, the electrolytic aqueous solution is contained in the positive electrode tank and the negative electrode tank, the positive electrode is in contact with the electrolytic aqueous solution contained in the positive electrode tank, the negative electrode is in contact with the electrolytic aqueous solution contained in the negative electrode tank, the heating mechanism heats one of the electrolytic aqueous solution contained in the positive electrode tank and the electrolytic aqueous solution contained in the negative electrode tank, the cooling mechanism cools the other one of the electrolytic aqueous solution contained in the positive electrode tank and the electrolytic aqueous solution contained in the negative electrode tank, and the circulation mechanism circulates the electrolytic aqueous solution between the positive electrode tank and the negative electrode tank. With this configuration, an ion concentration gradient is generated between the positive electrode tank and the negative electrode tank by the heating mechanism and the cooling mechanism, and thus electricity can be extracted from the positive electrode and the negative electrode. Also, with circulation of the electrolytic aqueous solution by the circulation mechanism, the ion concentration gradient is maintained, as a result of which power generation can be performed continuously.

Another characteristic configuration of a power generating device according to the present invention is that the power generating device includes a heat exchange mechanism, and the heat exchange mechanism performs heat exchange between the electrolytic aqueous solution delivered to the positive electrode tank by the circulation mechanism and the electrolytic aqueous solution delivered to the negative electrode tank by the circulation mechanism.

According to the characteristic configuration described above, the energy efficiency of the power generating device can be increased by effectively utilizing heat from the heating mechanism, and thus the characteristic configuration described above is preferable.

### Brief Description of the Drawings

FIG. 1 is a diagram showing an overview of a power generating device.
FIG. 2 is a diagram showing an overview of a testing apparatus for a power generation principle test.
FIG. 3 is a diagram showing an overview of a testing apparatus for a power generation principle test.
FIG. 4 is a graph showing the results of a power generation principle test.
FIG. 5 is a diagram showing an overview of a testing apparatus for a two-tank potential difference measurement test.
FIG. 6 is a graph showing the results of a two-tank potential difference measurement test.
FIG. 7 is a graph showing the results of a two-tank potential difference measurement test
FIG. 8 is a graph showing the results of a two-tank potential difference measurement test.
FIG. 9 is a graph showing the results of a two-tank potential difference measurement test.

### Best Mode for Carrying out the Invention

Hereinafter, an electrolytic solution, an electrolytic aqueous solution, and a power generating device will be described in detail. An electrolytic solution and an electrolytic aqueous solution according to the present embodiment contain a temperature responsive electrolyte and an oxidation-reduction active species (an oxidation-reduction reactive species).

There is no particular limitation on the temperature responsive electrolyte as long as an electrolyte whose affinity for ions and oxidation-reduction active species in the electrolyte varies by a change in temperature, for example, an electrolyte whose pKa or hydrophobicity varies according to temperature is used, but it is preferable to use a polymer electrolyte.

According to the present invention, the temperature responsive electrolyte is a molecule that has both a polar group and a hydrophobic group in the molecule, and also has a functional group (ionizable functional group) that can emit ions in an aqueous solution, such as a surfactant, poly(N-isopropylacrylamide), or a polypeptide (a protein or a peptide).

The ionizable functional group may be an acidic group that emits H⁺ or a basic group that may be positively charged, and can be selected as appropriate according to the intended use of the present invention. Examples of the acidic group include a sulfuric acid group, a carboxylic acid group, a phosphoric acid group, a phenolic hydroxide group, and the like. Examples of the basic group include an amino group, an imidazole group, a pyridyl group, and the like.

The temperature responsive electrolyte as described above may be produced by covalently attaching an ionizable functional group to a molecule that has both a polar group and a hydrophobic group in the molecule. Alternatively, the temperature responsive electrolyte may be produced by copolymerizing a monomer component that has an ionizable ionizing group, a monomer component that has a polar group, and a monomer component that has a hydrophobic group, or by copolymerizing a monomer component that has an ionizable ionizing group and a monomer component that has a polar group and a hydrophobic group.

Molecules that have both a polar group and a hydrophobic group in each molecule such as those of a surfactant, poly(N-isopropylacrylamide), or a polypeptide (a protein or a peptide) are temperature responsive, that is, the molecules are well dissolved and dispersed in water at low temperatures, but the molecules assemble, contract, aggregate, form into a gel, and precipitate due to hydrophobic interaction when they are heated to a given temperature or higher.

On the other hand, the degree of ionization (pKa) of the electrolyte varies reversibly according to the environment (polarity) in which the electrolyte is present or the distance between ions in the electrolyte. For example, sulfuric acid is mostly ionized in a high polarity aqueous solution, and has a structure of a high polarity sulfuric acid anion (a negative ion such as HSO₄⁻ or SO₄²⁻). However, when an organic solvent is added to decrease the polarity of the medium, the degree of ionization decreases, and a substantial portion thereof has a structure of a low polarity sulfuric acid (H₂SO₄). Also, a large number of carboxylic acid groups are densely collected on one molecule or polymer, or on a material, electrostatic repulsion occurs between adjacent carboxylate anions, and ions (negative ions such as RCOO-) become energetically unstable. Accordingly, the degree of ionization decreases, and the proportion of carboxylic acid (RCOOH) with no electric charge increases. Also, if protonated carboxylic acid is introduced into a low polarity polymer, the carboxylic acid is unlikely to be ionized, the degree of ionization decreases, and the proportion of carboxylic acid (RCOOH) with no electric charge increases.

In the temperature responsive electrolyte according to the present embodiment, an ionizable functional group (electrolyte) is combined with a combination of two properties of a polar group and a hydrophobic group, and an ion concentration gradient is generated by using a temperature difference.

With the temperature responsive electrolyte as described above, in a high temperature range, the molecules assemble, contract, aggregate, form into a gel, and precipitate, as a result of which the environment surrounding ions becomes hydrophobic (have a low polarity), or the distance between ions decreases, and thus the electrolyte is unlikely to be ionized. On the other hand, in a low temperature range, the molecules are dispersed, swell, and are dissolved, as a result of which the polarity of the surroundings increases, or the distance between ions increases, and thus the electrolyte is likely to be ionized. That is, a temperature responsive electrolyte that contains an acid (a functional group that may be negatively charged) such as sulfuric acid or carboxylic acid exhibits a low pKa value at low temperatures, but exhibits a high pKa value in a high temperature range. On the other hand, in a temperature responsive electrolyte that contains an amine-like base (a functional group that may be positively charged), the ammonium group that is a conjugate acid exhibits a high pKa value at low temperatures, but exhibits a low pKa value in a high temperature range.

A temperature responsive nano particle electrolyte was actually synthesized by copolymerizing acrylic acid that has carboxylic acid with N-isopropylacrylamide, and the pH was measured while changing the temperature. When the temperature was increased, the pH increased suddenly at a certain temperature. At this time, the observed pH gradient reached 0.1 K⁻¹ at maximum. According to the Nernst's equation, this corresponds to several ten mVK⁻¹. Also, the temperature dependence of the particle size of the nano particles was measured by a dynamic light scattering method. As a result, it was found that the nano particles underwent a phase transition and suddenly contracted at a temperature at which the pH started increasing. That is, it was found that the temperature responsive electrolyte (poly(N-isopropylacrylamide) copolymer nano particles that contained carboxylic acid) swelled at a low temperature, and thus a large number of carboxylic acid groups were ionized, but it contracted with an increase in temperature, and thus the degree of ionization of carboxylic acid decreased. This phenomenon is observed not only when an acidic temperature responsive electrolyte such as carboxylic acid is used but also when a basic temperature responsive electrolyte such as an amine group or an imidazole group is used.

When temperature responsive nano particle electrolytes were synthesized by copolymerizing, instead of acrylic acid, basic monomers that have an imidazole group (1-H-Imidazole-4-N-acryloylethanamine) and an amine group (N-[3-(Dimethylamino)propyl]methacrylamide) (DMAPM) with N-isopropylacrylamide so as to observe a temperature responsive pH change, the temperature responsive nano particle electrolytes exhibited a relatively high pH value at low temperatures below the phase transition point, but the pH suddenly dropped near the phase transition point when the temperature was increased. The pH gradient at this time also reached about 0.1 K⁻¹ at maximum, which corresponds to several ten mVK⁻¹ according to the Nernst's equation. A temperature responsive electrolyte obtained by copolymerizing N-[3-(Dimethylamino)propyl]methacrylamide that had an amine group instead of an imidazole group also exhibited a reduced pka in a high temperature range. Furthermore, when the temperature responsive nano particle electrolyte obtained by copolymerizing N-[3-(Dimethylamino)propyl]methacrylamide was subjected to pH titration using hydrochloric acid at different temperatures, the apparent point of neutralization shifted significantly around the phase transition point. That is, some of the dimethyl amino groups function as basic groups at a temperature lower than equal to the phase transition temperature, but do not function as basic groups at a temperature greater than or equal to the phase transition temperature because they are embedded in contracted polymer chains.

Conventionally, it is generally known that the pH of an electrolyte can be changed by changing the temperature, and the pH change of an ordinary electrolyte is about 0.01 K⁻¹ per 1°C. With the use of the temperature responsive electrolyte according to the present embodiment, it is possible to achieve a significant pH change, which has not been achieved by the existing electrolytes.

Most temperature responsive electrolytes have a phase transition point and suddenly undergo changes in the state around the phase transition temperature such as assembling, contracting, aggregating, gel forming, and precipitating. For this reason, the pH of an aqueous solution or the like that contains such a temperature responsive electrolyte can be changed suddenly with a small temperature change. Also, the phase transition temperature can be controlled not only by the polarity or ionic strength of a solution that contains the temperature responsive electrolyte or the concentration of the temperature responsive electrolyte, but also by the electrolyte density and the balance between hydrophilicity and hydrophobicity of the temperature responsive electrolyte.

Furthermore, the pH range and the temperature range that are changed can be controlled by controlling the type of electrolyte (strong acid, weak acid, weak base, strong base, or the like), the density, or the degree of assembly, contraction, aggregation, gel formation, and precipitation. That is, the pH level can be controlled from a very low pH level to a high pH level according to the molecular design or medium design to achieve the intended temperature responsiveness. For example, the swelling ratio due to temperature change can be adjusted by adjusting the amount of cross-linking monomer (copolymerization ratio of cross-linking monomer) such as N,N'-methylenebisacrylamide when the temperature responsive electrolyte polymer (nano particles) is synthesized. Also, the phase transition temperature can be adjusted by adjusting the amount of hydrophobic monomer (the copolymerization ratio of hydrophobic monomer) such as N-t-butylacrylamide when the temperature responsive electrolyte polymer (nano particles) is synthesized.

As reported in the article titled "Design Rationale of Thermally Responsive Microgel Particle Films that Reversibly Absorb Large Amounts of CO2: Fine Tuning the pKa of Ammonium Ions in the Particles" (Chemical Science, 2015, 6, 6112 to 6123), the actual pKa range and pKa change of amine-containing gel particles can be tuned as desired by designing the type of amine, pH during polymerization, crosslink density, particle size, and hydrophobic functional group.

Likewise, as reported in the article titled "Rational Design of Synthetic Nanoparticles with a Large Reversible Shift of Acid Dissociation Constants: Proton Imprinting in Stimuli Responsive Nanogel Particles" (ADVANCED MATERIALS, 2014, 26, 3718 to 3723), the actual pKa range and pKa change of carboxylic acid-containing gel particles can be tuned as desired by designing the type of carboxylic acid, pH during polymerization, crosslink density, particle size, and hydrophobic functional group.

### Oxidation-Reduction Active Species

In the oxidation-reduction active species used in the present embodiment, both the oxidized form and the reduced form are soluble in the aqueous solution during the process of oxidation-reduction by exchange of electrons, and hydrogen ions (protons) are contained in the oxidation-reduction reaction system. Then, the oxidation-reduction active species used in the present embodiment forms an equilibrium state (oxidation-reduction equilibrium) in the electrolytic solution (electrolytic aqueous solution).

Also, as the oxidation-reduction active species according to the present embodiment, oxidation-reduction active species whose oxidation-reduction potential varies according to the pH is used. Alternatively, oxidation-reduction active species in which the interaction with the temperature responsive electrolyte varies according to the temperature may also be used. The interaction may be any one or a combination of a hydrophobic interaction, an electrostatic interaction, and a hydrogen bond.

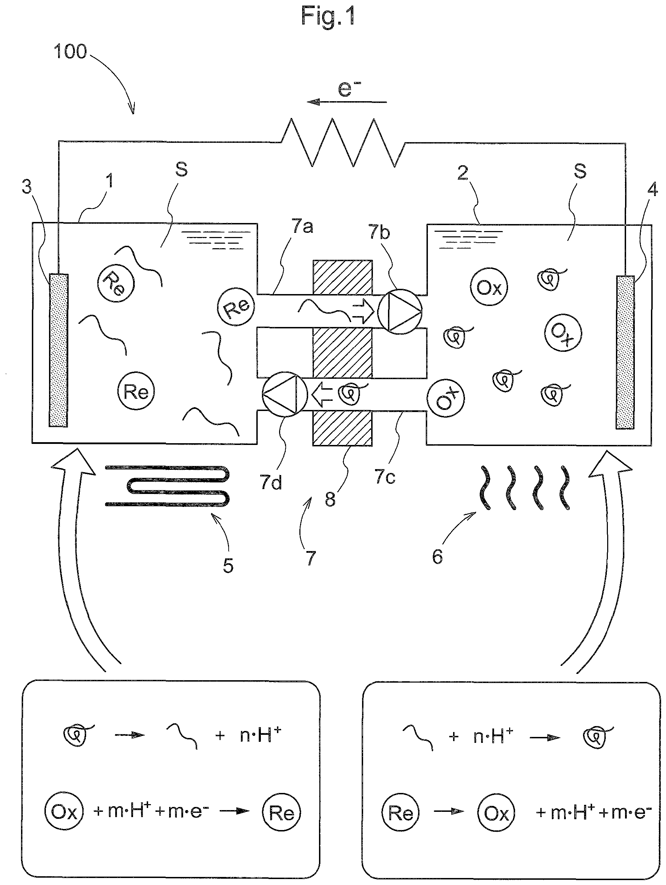

In the present embodiment, the term "oxidized form" refers to a state in which the oxidation-reduction active species is oxidized in the oxidation-reduction and functions as a so-called oxidizing agent. The term "reduced form" refers to a state in which the oxidation-reduction active species is reduced in the oxidation-reduction reaction and functions as a so-called reducing agent. In FIG. 1, which will be described later, the oxidized form is represented by "Ox", and the reduced form is represented by "Re".

In the present embodiment, as the oxidation-reduction active species, an anthraquinone derivative, a nicotinamide derivative, N-substituted nicotinamide, a riboflavin derivative, or a proflavine derivative is used. Also, methylene blue, N,N,N',N'-tetramethyl-p-phenylenediamine, dithiothreitol, a ferrocyanide compound, N1-ferrocenylmethyl-N1,N1,N2,N2,N2-pentamethylpropane- 1,2-diaminium dibromide, methylviologen, naphthoquinone, menadione, a hydroquinone derivative, or the like is also preferably used as the oxidation-reduction active species.

### Anthraquinone Derivative

As the anthraquinone derivative, for example, sulfated anthraquinone (hereinafter also referred to simply as "AQDS") can be used. Sulfated anthraquinone is a substance hydrophilized by introducing a sulfone group into anthraquinone. Through hydrophilization, the oxidation-reduction active species can be uniformly dispersed in the electrolytic solution, and it is therefore preferable. That is, the hydrophilized anthraquinone derivative can be used preferably as the oxidation-reduction active species.

The structure of sulfated anthraquinone is represented by chemical formula 2, and the oxidation-reduction reaction formula of sulfated anthraquinone is represented by chemical formula 3. In an environment with a low pH level, the reduction reaction of the oxidized form (the left-hand side of the oxidation-reduction reaction formula) proceeds, the equilibrium shifts to the right, and protons and electrons are consumed. On the other hand, in an environment with a high pH level, the oxidation reaction of the reduced form (the right-hand side of the oxidation-reduction reaction formula) proceeds, the equilibrium shifts to the left, and protons and electrons are emitted.

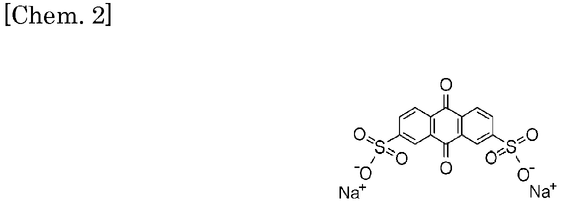

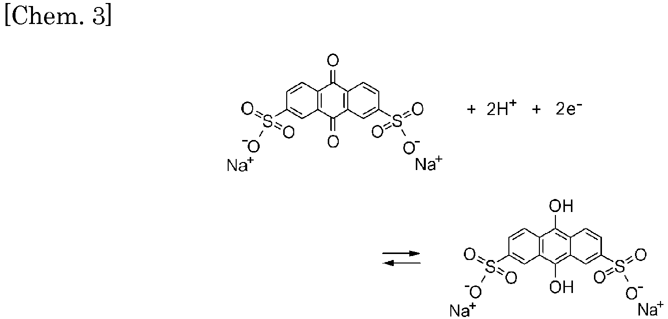

### Methylene Blue

The structure of methylene blue (hereinafter also referred to simply as "MB") is represented by chemical formula 4, and the oxidation-reduction reaction formula of methylene blue is represented by chemical formula 5. In an environment with a low pH level, the reduction reaction of the oxidized form (the left-hand side of the oxidation-reduction reaction formula) proceeds, the equilibrium shifts to the right, and protons and electrons are consumed. On the other hand, in an environment with a high pH level, the oxidation reaction of the reduced form (the right-hand side of the oxidation-reduction reaction formula) proceeds, the equilibrium shifts to the left, and protons and electrons are emitted.

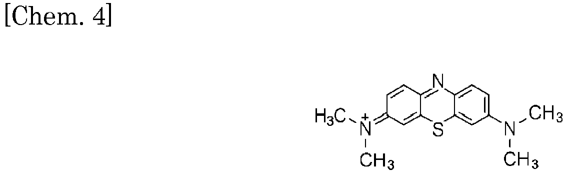

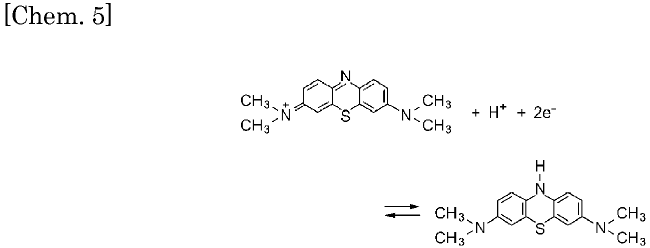

### Hydroquinone Derivative

As the hydroquinone derivative that does not precipitate in the electrolytic aqueous solution, for example, hydroquinone or a derivative thereof can be used. Hereinafter, an example will be described in which hydroquinone is used. In the aqueous solution, a portion of hydroquinone dissociates into benzoquinone and forms an equilibrium state (acid dissociation equilibrium) represented by chemical formula 6.

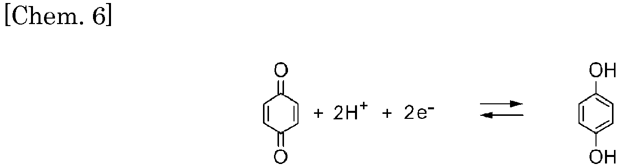

In an environment with a low pH level, the reduction reaction of the benzoquinone (oxidized form) proceeds, the equilibrium shifts to the right, and protons and electrons are consumed. On the other hand, in an environment with a high pH level, the oxidation reaction of the hydroquinone (reduced form) proceeds, the equilibrium shifts to the left, and protons and electrons are emitted.

As the hydroquinone derivative, it is possible to use a compound that is represented by chemical formula 7 or 8 given below, and is water soluble both before and after the acid dissociation equilibrium reaction or does not precipitate in the electrolytic aqueous solution. Here, R₂ to R₆ represent a hydrogen group, a hydrocarbon group such as an alkyl group, an alkenyl group or a phenyl group, a hydroxy group, an alkoxy group, an amine group, a carboxy group, a sulfo group, a nitro group, a cyan group, or a thiol group.

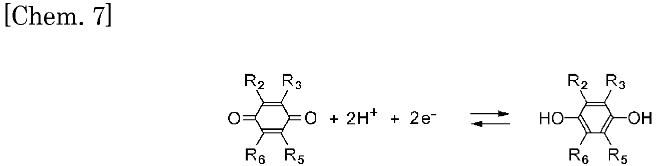

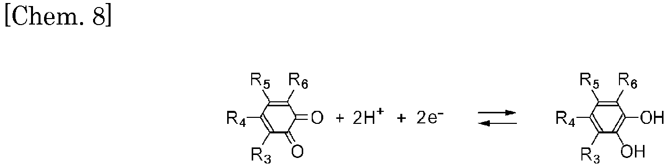

### Power Generating Device

Next, a description will be given of a power generating device in which the electrolytic solution or electrolytic aqueous solution (hereinafter also referred to simply as "electrolytic solution or the like") described above is used, with reference to FIG. 1. A power generating device 100 according to the present embodiment includes a positive electrode tank 1, a negative electrode tank 2, a positive electrode 3, a negative electrode 4, a cooling mechanism 5, a heating mechanism 6, a circulation mechanism 7, and a heat exchange mechanism 8.

An electrolytic solution S or an electrolytic aqueous solution S (an electrolytic solution S or the like) as described above is contained in the positive electrode tank 1 and the negative electrode tank 2. The positive electrode 3 and the negative electrode 4 are, for example, electrodes made of carbon. The positive electrode 3 is immersed in the electrolytic solution S or the like contained in the positive electrode tank 1. The negative electrode 4 is immersed in the electrolytic solution S or the like contained in the negative electrode tank 2.

The cooling mechanism 5 cools the electrolytic solution S or the like contained in the positive electrode tank 1. That is, the cooling mechanism 5 cools the electrolytic solution S or the like that is present in the vicinity of the positive electrode 3. For example, the cooling mechanism 5 is a heat exchanger that exchanges heat between the electrolytic solution S or the like contained in the positive electrode tank 1 and seawater.

The heating mechanism 6 heats the electrolytic solution S or the like contained in the negative electrode tank 2. That is, the heating mechanism 6 heats the electrolytic solution S or the like that is present in the vicinity of the negative electrode 4. For example, the heating mechanism 6 is a heat exchanger that exchanges heat between the electrolytic solution S or the like contained in the negative electrode tank 2 and high-temperature water from a plant, an internal combustion engine, a fuel cell, or the like.

The circulation mechanism 7 is a mechanism that circulates the electrolytic solution S or the like between the positive electrode tank 1 and the negative electrode tank 2. The circulation mechanism 7 includes a first flow path 7a, a first pump 7b, a second flow path 7c, and a second pump 7d. The first flow path 7a and the second flow path 7c are flow paths that allow the electrolytic solution S or the like to flow therethrough and connect the positive electrode tank 1 and the negative electrode tank 2. The first pump 7b is a pump that is provided in the first flow path 7a and pumps the electrolytic solution S or the like contained in the positive electrode tank 1 to the negative electrode tank 2. The second pump 7d is a pump that is provided in the second flow path 7c and pumps the electrolytic solution S or the like contained in the negative electrode tank 2 to the positive electrode tank 1.

The heat exchange mechanism 8 is a heat exchanger that exchanges heat between the electrolytic solution S or the like delivered to the positive electrode tank 1 by the circulation mechanism 7 and the electrolytic solution S delivered to the negative electrode tank 2 by the circulation mechanism 7. Specifically, the heat exchange mechanism 8 exchanges heat between the electrolytic solution S or the like that flows through the first flow path 7a and the electrolytic solution S or the like that flows through the second flow path 7c.

### Operations of Power Generating Device

The operations of the power generating device 100 described above will be described. An example will be described below in which the ionizable functional groups contained in the temperature responsive electrolyte are acids such as sulfuric acid and carboxylic acid (functional groups that may be negatively charged).

The heating mechanism 6 heats the electrolytic solution S or the like contained in the negative electrode tank 2 so as to increase the temperature of the electrolytic solution S or the like, in particular, to a temperature higher than the phase transition temperature of the temperature responsive electrolyte. In response thereto, the temperature responsive electrolyte (molecules) undergoes dehydrating, assembling, contracting, aggregating, gel forming, precipitating, and the like. As a result, the environment surrounding the functional groups of the temperature responsive electrolyte becomes hydrophobic (have a low polarity), or the distance between functional groups decreases, which makes it difficult for the functional groups to ionize. That is, the pKa value of the temperature responsive electrolyte increases, and the concentration of hydrogen ions (protons) in the negative electrode tank 2 decreases. Accordingly, the pH of the electrolytic solution S or the like contained in the negative electrode tank 2 increases. At the same time, the interaction between the temperature responsive electrolyte and either one or both of the oxidized form and the reduced form of the oxidation-reduction active species may change.

As a result, the oxidation-reduction equilibrium of the oxidation-reduction active species represented by chemical formula 3 or the like shifts to the left. That is, the oxidation reaction of the reduced form proceeds, and protons and electrons are emitted. The electrons emitted from the oxidation-reduction active species are drawn to the outside from the negative electrode 4.

The cooling mechanism 5 cools the electrolytic solution S or the like contained in the positive electrode tank 1 so as to decrease the temperature of the electrolytic solution S or the like to a low temperature (lower than the temperature of the negative electrode tank 2), in particular, to a temperature lower than the phase transition temperature of the temperature responsive electrolyte. In response thereto, the temperature responsive electrolyte (molecules) are dispersed, swell, are dissolved, and the like. As a result, the polarity of the environment surrounding the functional groups of the temperature responsive electrolyte increases, or the distance between functional groups increases, which makes it easy for the functional groups to ionize. That is, the pKa value of the temperature responsive electrolyte decreases, and the concentration of hydrogen ions (protons) in the positive electrode tank 1 increases. Accordingly, the pH of the electrolytic solution S or the like contained in the positive electrode tank 1 decreases.

As a result, the oxidation-reduction equilibrium of the oxidation-reduction active species represented by chemical formula 3 or the like shifts to the right. That is, the reduction reaction of the oxidized form proceeds by using protons in the surroundings and electrons supplied from the positive electrode 3. That is, the electrons from the negative electrode 4 are consumed in the positive electrode tank 1.

Through the reactions described above, in the positive electrode tank 1, the concentration of the temperature responsive electrolyte (molecules) that has been dispersed, swollen, been dissolved, and the like and the concentration of the reduced form of the oxidation-reduction active species increase. In the negative electrode tank 2, the concentration of the temperature responsive electrolyte (molecules) that has undergone assembling, contracting, aggregating, gel forming, precipitating, and the like and the concentration of the oxidized form of the oxidation-reduction active species increase. As a result of the circulation mechanism 7 circulating the electrolytic solution S or the like between the positive electrode tank 1 and the negative electrode tank 2, the concentrations of these substances are balanced, the above-described reactions proceed continuously, and power generation by the power generating device 100 is performed continuously.

As described above, the power generating device 100 according to the present embodiment is a power generating device that performs power generation by using the electrolytic solution or the like described above, the power generating device 100 including a positive electrode 3, a negative electrode 4, a heating mechanism 6, and a cooling mechanism 5, and the positive electrode 3 and the negative electrode 4 being immersed in the electrolytic solution or the like. The heating mechanism 6 heats the electrolytic solution or the like that is present in the vicinity of the negative electrode 4 to a temperature higher than the phase transition temperature of the temperature responsive electrolyte. The cooling mechanism 5 cools the electrolytic solution or the like that is present in the vicinity of the positive electrode 3 to a temperature lower than the phase transition temperature of the temperature responsive electrolyte.

### Operations of Power Generating Device (Another Embodiment)

Here, an example will be described in which the ionizable functional groups of the temperature responsive electrolyte are amine-like basic groups (functional groups that may be positively charged). In this case, the ammonium group in the temperature responsive electrolyte has a high pKa value at low temperatures, but has a low pKa value in a high temperature range. In the case where the temperature responsive electrolyte described above is used in the power generating device 100, the positions of the cooling mechanism 5 and the heating mechanism 6 are swapped. That is, the power generating device 100 is configured such that the cooling mechanism 5 cools the electrolytic solution S or the like contained in the negative electrode tank 2, and the heating mechanism 6 heats the electrolytic solution S or the like contained in the positive electrode tank 1.

The cooling mechanism 5 cools the electrolytic solution S or the like contained in the negative electrode tank 2 so as to decrease the temperature of the electrolytic solution S or the like to a low temperature, in particular, to a temperature lower than the phase transition temperature of the temperature responsive electrolyte. In response thereto, the temperature responsive electrolyte (molecules) is dispersed, swells, is dissolved, and the like. As a result, the polarity of the environment surrounding the functional groups of the temperature responsive electrolyte increases, or the distance between functional groups increases, which makes it easy for the functional groups to ionize. That is, the pKa value of the temperature responsive electrolyte increases, and the concentration of hydrogen ions (protons) in the negative electrode tank 2 decreases. Accordingly, the pH of the electrolytic solution S or the like contained in the negative electrode tank 2 increases.

As a result, the oxidation-reduction equilibrium of the oxidation-reduction active species represented by chemical formula 3 or the like shifts to the left in chemical formula 3 or the like. That is, the oxidation reaction of the reduced form proceeds, and protons and electrons are emitted. The electrons emitted from the oxidation-reduction active species are drawn to the outside from the negative electrode 4.

The heating mechanism 6 heats the electrolytic solution S or the like contained in the positive electrode tank 1 so as to increase the temperature of the electrolytic solution S or the like to a high temperature (higher than the temperature of the negative electrode tank 2), in particular, to a temperature higher than the phase transition temperature of the temperature responsive electrolyte. In response thereto, the temperature responsive electrolyte (molecules) undergoes assembling, contracting, aggregating, gel forming, precipitating, and the like. As a result, the environment surrounding the functional groups of the temperature responsive electrolyte becomes hydrophobic (have a low polarity), or the distance between functional groups decreases, which makes it difficult for the functional groups to ionize. That is, the pKa value of the temperature responsive electrolyte decreases, and the concentration of hydrogen ions (protons) in the positive electrode tank 1 increases. Accordingly, the pH of the electrolytic solution S or the like contained in the positive electrode tank 1 decreases.

As a result, the oxidation-reduction equilibrium of the oxidation-reduction active species represented by chemical formula 3 or the like shifts to the right in chemical formula 3. That is, the reduction reaction of the oxidized form proceeds by using protons in the surroundings and electrons supplied from the positive electrode 3. That is, the electrons from the negative electrode 4 are consumed in the positive electrode tank 1.

Through the reactions described above, in the positive electrode tank 1, the concentration of the temperature responsive electrolyte (molecules) that has undergone assembling, contracting, aggregating, gel forming, precipitating, and the like and the concentration of the reduced form of the oxidation-reduction active species increase. In the negative electrode tank 2, the concentration of the temperature responsive electrolyte (molecules) that has been dispersed, swollen, been dissolved, and the like and the concentration of the oxidized form of the oxidation-reduction active species increase. As a result of the circulation mechanism 7 circulating the electrolytic solution S or the like between the positive electrode tank 1 and the negative electrode tank 2, the concentrations of these substances are balanced, the above-described reactions proceed continuously, and power generation by the power generating device 100 is performed continuously.

As described above, the power generating device 100 according to the present embodiment is a power generating device that performs power generation by using the electrolytic solution or the like described above, the power generating device 100 including a positive electrode 3, a negative electrode 4, a heating mechanism 6, and a cooling mechanism 5, and the positive electrode 3 and the negative electrode 4 being immersed in the electrolytic solution or the like. The heating mechanism 6 heats the electrolytic solution or the like that is present in the vicinity of the positive electrode 3 to a temperature higher than the phase transition temperature of the temperature responsive electrolyte. The cooling mechanism 5 cools the electrolytic solution or the like that is present in the vicinity of the negative electrode 4 to a temperature lower than the phase transition temperature of the temperature responsive electrolyte.

### Test 1: Power Generation Principle Test 1

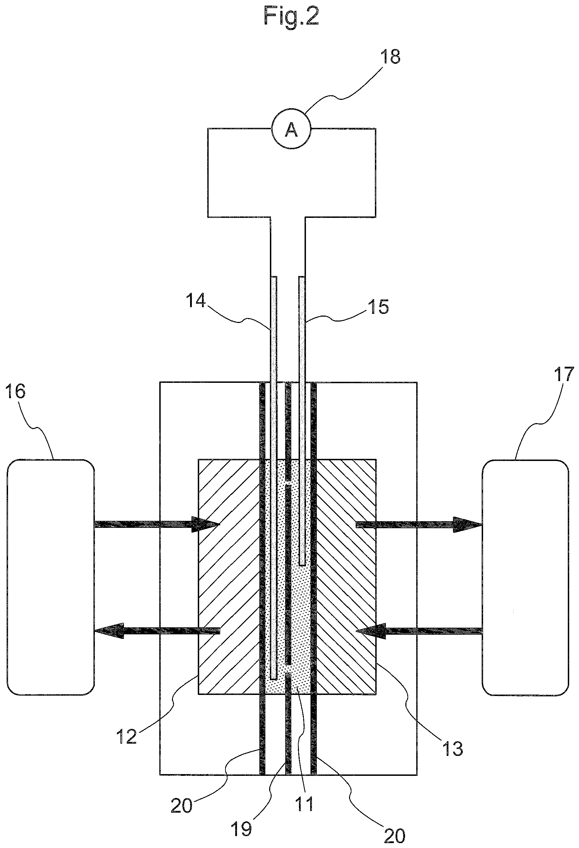

In order to confirm that power generation can be performed by using the electrolytic solution according to the present embodiment, a power generation principle test was performed by using test equipment shown in FIG. 2.

As shown in FIG. 2, the test equipment includes an aqueous solution tank 11, a cooling water circulation tank 12, a high-temperature water circulation tank 13, a low-temperature side electrode 14, a high-temperature side electrode 15, a low-temperature heat source 16, a high-temperature heat source 17, a current/voltage meter 18 (2401 Source Meter available from Keithley), a baffle plate 19, and heat transfer plates 20. The aqueous solution tank 11 is filled with a test sample.

The low-temperature side electrode 14 and the high-temperature side electrode 15 are brought into contact with the test sample contained in the aqueous solution tank 11. The low-temperature side electrode 14 is a platinum wire with a thickness of 1 mm, and a portion that is brought into contact with the test sample has a length of 22 mm. The high-temperature side electrode 15 is a platinum wire with a thickness of 1 mm, and a portion that is brought into contact with the test sample has a length of 18 mm. The low-temperature side electrolytic aqueous solution tank and the high-temperature side aqueous solution tank are partially separated by the baffle plate with a thickness of 0.2 mm, and are designed such that a thermal convection is effectively maintained. The low-temperature side electrolytic aqueous solution tank and the high-temperature side aqueous solution tank have a thickness of 3 mm, and have a cylindrical shape with a diameter of 30 mm. The current meter 18 is connected between the low-temperature side electrode 14 and the high-temperature side electrode 15.

The low-temperature side water tank 12 is in contact with the water tank via a heat transfer plate with a thickness of 0.1 mm, and water with a controlled temperature is supplied from the low-temperature heat source 16. The high-temperature side water tank 13 is in contact with the water tank via a heat transfer plate with a thickness of 0.1 mm, and water with a controlled temperature is supplied from the high-temperature heat source 17. With this configuration, in the aqueous solution tank 11, the test sample in the vicinity of the low-temperature side electrode 14 is cooled, and the test sample in the vicinity of the high-temperature side electrode 15 is heated, as a result of which a temperature difference (temperature gradient) occurs in the test sample contained in the aqueous solution tank 11. Also, in the aqueous solution tank 11, due to convection, the test sample circulates between the low-temperature side electrode 14 and the high-temperature side electrode 15.

By using the test equipment configured as described above, the magnitudes of voltage and electric current generated between the low-temperature side electrode 14 and the high-temperature side electrode 15 were measured. Also, the temperature difference may also be measured by inserting a thermocouple with a diameter of 1 mm instead of the electrodes.

### Test Sample

An aqueous solution as shown below was prepared as the test sample (electrolytic solution), and subjected to a test using the test equipment described above.

| | |
|---|---|
| Sulfated anthraquinone | 1 mmol/L |
| Reducing agent (sodium dithionite) | 0.5 mmol/L |
| KCl | 30 mmol/L |
| NaOH | 2 mmol/L |
| Temperature responsive electrolyte (Nps (COO⁻)) | 4 mmol/L |

Here, as the temperature responsive electrolyte (Nps), a temperature responsive nano particle electrolyte obtained by copolymerizing acrylic acid that had carboxylic acid with N-isopropylacrylamide was used. The concentration of carboxylic acid in the electrolyte was adjusted to be 4 mmol/L. As the nano particle electrolyte, specifically, nano particles obtained by copolymerizing 93 mol% of N-isopropylacrylamide, 5 mol% of acrylic acid, and 2 mol% of N,N'-methylenebisacrylamide as a cross-linking agent were used. The particles (hereinafter also referred to as "Nps") were produced in the following manner.

Acrylic acid (AAc), N-Isopropylacrylamide (NIPAm), N,N'-Methylenebisacrylamide (BIS), and sodium dodecyl sulfate (SDS) were dissolved in 300 mL of MiliQ water such that the total monomer concentration was 312 mM. The composition was NIPAm 93 mol%, BIS 2 mol%, AAc 5 mol%, SDS 6.21 mM, V-501 19.3 mg/1.96mL DMSO. The pH before polymerization was adjusted to 3.5 by using 1 M HCl and 1 M NaOH. After that, N₂ was bubbled in a mixed solution for 30 minutes. An initiator V-501 was dissolved in DMSO, and added to the mixed solution so as to initiate polymerization. The polymerization was performed by stirring in a N₂ atmosphere at 70°C for three hours. The solution after reaction was purified through dialysis by exchanging water using a dialysis membrane (MWCO 12,000 to 14,000) such that the amount of surfactant was 0.25% or less of the total monomer concentration. Counter anions were removed using a cation exchange resin (Muromac C1002-H). The cation exchange resin was separated through filtration. The concentration of Nps aqueous solution was determined from the weight of Nps by freeze drying 5 mL of the dialyzed aqueous solution. The amount of AAc introduced was quantified through acid-base neutralization titration, and from the titration curve, the pKa value when the electrolyte swelled and the pKa value when the electrolyte contracted were obtained.

Sulfated anthraquinone was mixed with a reducing agent (sodium dithionite) in advance so as to obtain a mixture with the reduced form at a ratio of 1:1, before use.

It was confirmed that when water that had the same temperature (20°C) was allowed to flow through the cooling water circulation tank 12 and the high-temperature water circulation tank 13 so as to not generate a temperature gradient in the aqueous solution tank 11, the voltage was 40 mV or less, and the electric current was 0.003 µA or less. It was also confirmed that when water at a temperature of about 10°C was allowed to flow through the cooling water circulation tank 12, and water at a temperature of about 70°C was circulated through the high-temperature water circulation tank 13, the temperature of the electrolyte on the low temperature side in the aqueous solution tank 11 was 20 °C, and the temperature of the electrolyte on the high temperature side in the aqueous solution tank 11 was 58°C. In this state, potential difference measurement was performed using platinum electrodes, and a potential difference of 295 mV was observed. Also, electric current measurement was performed, and a maximum current of about 0.2 µA was observed. The value decreased gradually, and remained steady at 0.063 µA. From the results described above, it was confirmed that it is possible to perform power generation by using an electrolytic solution that contains a temperature responsive electrolyte and sulfated anthraquinone. The reason that the current value decreased is considered to be that the convection was suppressed by the structure of the baffle plate.

### Test 2: Power Generation Principle Test 2

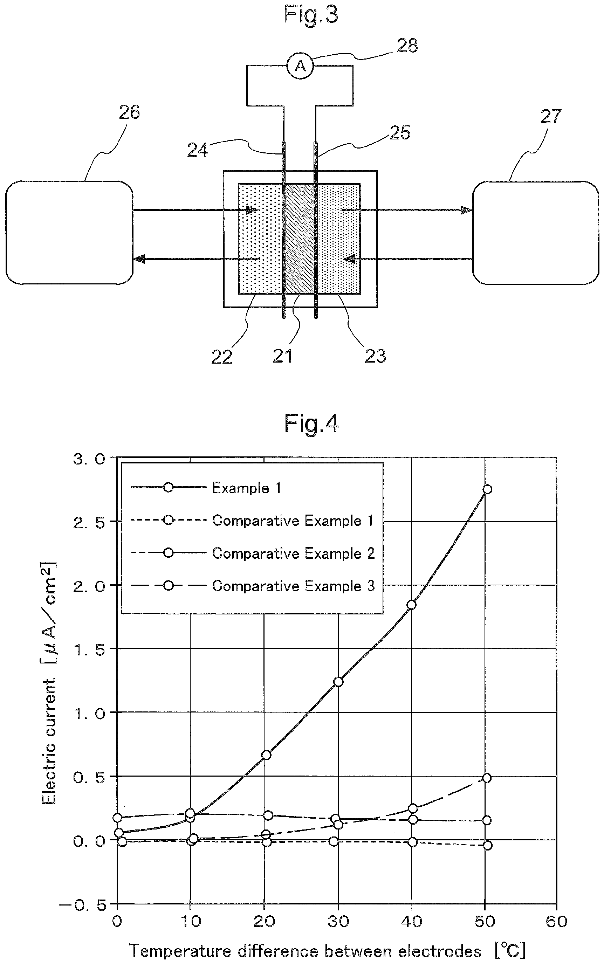

In order to confirm that power generation can be performed by using the electrolytic solution according to the present embodiment, a power generation principle test was performed by using test equipment shown in FIG. 3.

As shown in FIG. 3, the test equipment includes an aqueous solution tank 21, a low-temperature side water tank 22, a high-temperature side water tank 23, a low-temperature side electrode 24, a high-temperature side electrode 25, a low-temperature heat source 26, a high-temperature heat source 27, and a current meter 28. The aqueous solution tank 21 is filled with a test sample.

The low-temperature side electrode 24 and the high-temperature side electrode 25 are brought into contact with the test sample contained in the aqueous solution tank 21. The low-temperature side electrode 24 and the high-temperature side electrode 25 are thin platinum plates with a thickness of 0.1 µm (Ti substrates with a thickness of 0.5 mm), and a portion that is brought into contact with the test sample has a size of 25 mm × 25 mm. The distance between the low-temperature side electrode 24 and the high-temperature side electrode 25 is 6.4 mm. The current meter 28 is connected between the low-temperature side electrode 24 and the high-temperature side electrode 25.

The low-temperature side water tank 22 is in contact with the low-temperature side electrode 24, and water with a controlled temperature is supplied from the low-temperature heat source 26. The high-temperature side water tank 23 is in contact with the high-temperature side electrode 25, and water with a controlled temperature is supplied from the high-temperature heat source 27. With this configuration, in the aqueous solution tank 21, the test sample in the vicinity of the low-temperature side electrode 24 is cooled, and the test sample in the vicinity of the high-temperature side electrode 25 is heated, as a result of which a temperature difference (temperature gradient) occurs in the test sample contained in the aqueous solution tank 21.

By using the test equipment configured as described above, a temperature difference was generated in the test sample contained in the aqueous solution tank 21 by controlling the temperatures of the low-temperature heat source 26 and the high-temperature heat source 27, and the magnitude of electric current flowing through the current meter 28 was measured. The temperature difference used to measure electric current was set to six different levels: 0°C, 10°C, 20°C, 30°C, 40°C, and 50°C.

### Test Sample

Aqueous solutions as shown in the table below were prepared as test samples, and subjected to a test using the test equipment described above.

**[Table 1]**

| | Component | Concentration |
|---|---|---|
| Example 1 | Quinhydrone | 0.005 mol/L |
| | Sodium sulfate | 0.1 mol/L |
| | Sodium hydroxide | 0.0005 mol/L |
| | Temperature responsive electrolyte | 2.5 wt% |
| Comparative Example 1 | Sodium sulfate | 0.1 mol/L |
| | Sodium hydroxide | 0.0005 mol/L |
| Comparative Example 2 | Quinhydrone | 0.005 mol/L |
| | Sodium sulfate | 0.1 mol/L |
| | Sodium hydroxide | 0.0005 mol/L |
| Comparative Example 3 | K₃[Fe(CN)₆] | 0.005 mol/L |
| | Sodium sulfate | 0.1 mol/L |

Here, as the temperature responsive electrolyte, a temperature responsive nano particle electrolyte obtained by copolymerizing acrylic acid that had carboxylic acid with N-isopropylacrylamide was used. More specifically, nano particles obtained by copolymerizing 68 mol% of N-isopropylacrylamide, 10 mol% of acrylic acid, 20 mol% of highly hydrophobic N-t-butylacrylamide, and 2 mol% of N,N'-methylenebisacrylamide as a cross-linking agent were used. The particles were prepared in the following manner.

N-isopropylacrylamide in an amount of 120 mg, N-t-butylacrylamide in an amount of 38.4 mg, acrylic acid in an amount of 11 µL, N,N'-methylenebisacrylamide in an amount of 4.6 mg, and sodium dodecyl sulfate in an amount of 17.4 mg were dissolved in 30 mL of ultra-pure water, and the solution was placed in a 100 mL eggplant flask, and the eggplant flask was hermetically sealed with a septum. The temperature was increased to 70°C using an oil bath, and in this state, the solution was stirred until uniform by using a magnetic stirrer. After the solution was uniform, two needles were inserted into the septum, with the tip of one of the needles being placed under the liquid surface and the tip of the other needle being placed above the liquid surface, and nitrogen was slowly bubbled into the needle placed under the liquid surface from the outside so as to perform degassing for 30 minutes. Then, 5.88 mg of 4,4'-Azobis (4 cyano-valeic acid) was dissolved in 0.6 ml of dimethyl sulfoxide, and the resultant was added to the solution using a needle. All of needles other than the one connected to nitrogen were removed, and the solution was reacted in a nitrogen atmosphere at 70°C for three hours. The reaction was stopped by opening the septum, and the reaction solution was placed in a dialysis tube with 10,000 Da MWCO, and dialysis was performed for three days while repeatedly replacing a large amount of water, so as to remove the surfactant and unreacted monomers.

Quinhydrone is a mixture of p-benzoquinone and p-hydroquinone.

The results of the power generation principle test are plotted in the graph of FIG. 4. In Comparative Examples 1 and 2, the electric current did not increase along with an increase in temperature difference between electrodes. In contrast, in Example 1, the electric current increased significantly along with an increase in temperature difference between electrodes. In Comparative Example 3, the electric current only reached 0.5 µA/cm² at a temperature difference between electrodes of 50°C. In contrast, in Example 1, an electric current of 2.7 µA/cm² was obtained at a temperature difference between electrodes of 50°C, which was five times or more that of Comparative Example 3. From the results described above, it was confirmed that it is possible to perform power generation by using an electrolytic aqueous solution that contains a temperature responsive electrolyte and hydroquinone.

### Test 3: Two-Tank Potential Difference Measurement Test 1

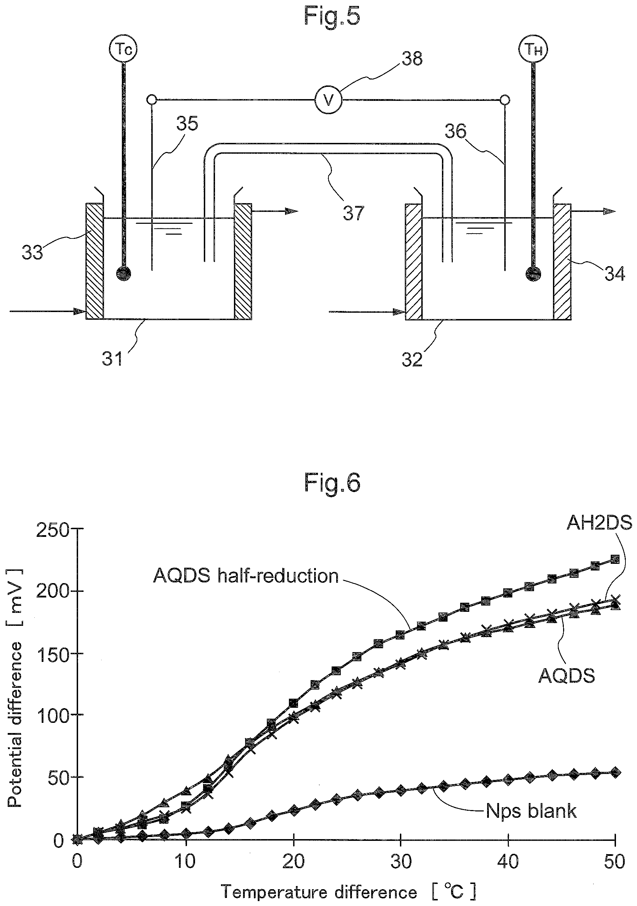

In order to confirm that power generation can be performed by using a configuration in which two tanks are used, as with the power generating device 100 according to the present embodiment, a two-tank potential difference measurement test was performed by using test equipment shown in FIG. 5.

As shown in FIG. 5, the test equipment includes a low-temperature side tank 31, a the high-temperature side tank 32, a low-temperature bath 33, a high-temperature bath 34, a low-temperature side electrode 35, a high-temperature side electrode 36, and a salt bridge 37. A test sample is contained in the low-temperature side tank 31 and the high-temperature side tank 32. Then, the test sample contained in the low-temperature side tank 31 and the test sample contained in the high-temperature side tank 32 are electrically connected by the salt bridge 37.

The low-temperature side electrode 35 and the high-temperature side electrode 36 are immersed in the test sample. As the low-temperature side electrode 35 and the high-temperature side electrode 36, glassy carbon electrodes are used. A voltage meter 38 is connected between the low-temperature side electrode 35 and the high-temperature side electrode 36. Also, as the salt bridge 37, acrylamide hydrogel (containing KCl) crosslinked with methylenebisacrylamide is used.

The low-temperature side tank 31 is immersed in water contained in the low-temperature bath 33. Water with a controlled temperature is supplied to the low-temperature bath 33 from a low-temperature heat source (not shown). The high-temperature side tank 32 is immersed in water contained in the high-temperature bath 34. Water with a controlled temperature is supplied to the high-temperature bath 34 from a high-temperature heat source (not shown). With the configuration described above, the test sample contained in the low-temperature side tank 31 is cooled, and the test sample contained in the high-temperature side tank 32 is heated, as a result of which a temperature difference occurs between the test sample contained in the low-temperature side tank 31 and the test sample contained in the high-temperature side tank 32.

By using the test equipment configured as described above, a temperature difference was generated between the test sample contained in the low-temperature side tank 31 and the test sample contained in the high-temperature side tank 32, and the magnitude of voltage generated between the low-temperature side electrode 35 and the high-temperature side electrode 36 was measured using the voltage meter 38. The voltage was measured by changing the temperature difference from 0°C to 50° C.

### Test Sample

Aqueous solutions as shown in the table below were prepared as test samples, and subjected to a test using the test equipment described above.

**[Table 2]**

| Sample name | Nps Blank (mmol/L) | AQDS (mmol/L) | AQDS half-reduced (mmol/L) | AH2DS (mmol/L) |
|---|---|---|---|---|
| AQDS | 0 | 1.0 | 1.0 | 1.0 |
| DITH | 0 | 0 | 0.5 | 1.0 |
| Nps (COO⁻) | 4.0 | 4.0 | 4.0 | 4.0 |
| NaOH | 2.0 | 2.0 | 2.0 | 2.0 |
| KCl | 30.0 | 30.0 | 30.0 | 30.0 |

The samples were prepared by mixing AQDS (sulfated anthraquinone), DITH (sodium dithionite), Nps (the same nano particles (temperature responsive electrolyte) as those used in test 1), sodium hydroxide, and potassium chloride at the ratio shown in Table 2. Nps concentration is indicated by carboxylic acid equivalent.

The sample "Nps Blank" is a comparative sample that does not contain oxidation-reduction active species. The sample "AQDS" contains Nps in an excess amount (four times) relative to AQDS. The sample "AQDS half-reduced" is a sample obtained by mixing a reducing agent (DITH) that reduces a half amount of AQDS. The sample "AH2DS" is a sample obtained by mixing a reducing agent (DITH) that reduces a full amount of AQDS.

The results of the two-tank potential difference measurement test 1 are plotted in the graph of FIG. 6. In all of the four samples, the potential difference increased significantly at a temperature difference of 10 ° C to 20 ° C. This range is the range in which the electrolytic solution has a temperature of 30 ° C to 40 ° C, and the pH varies significantly due to phase transition of the nano particles, and thus it is considered that the phase transition of the nano particles directly affects the potential difference.

Also, the results of the generation of potential difference also vary in proportion to the difference in the compositions of the samples. In the sample "Nps Blank", the potential difference increased with an increase in temperature difference, but the potential difference was 54.95 mV (50° C) at maximum. In contrast, the samples obtained by mixing AQDS, which is the oxidation-reduction active species, exhibited a potential difference much larger than that of the sample "Nps Blank". The sample "AQDS half-reduced" exhibited a potential difference of 225.41 mV, which was the highest in the test, at a temperature difference of 50°C. The sample "AQDS" exhibited a potential difference of 189.50 mV at a temperature difference of 50°C, and the sample "AH2DS" exhibited a temperature difference of 193.33 mV at a temperature difference of 50°C.

It was confirmed, from the test described above, that a larger potential difference can be generated when the temperature responsive electrolyte and the oxidation-reduction active species (AQDS) are used together than when the temperature responsive electrolyte is used alone for power generation (the sample "Nps Blank").

### Test 4: Two-Tank Potential Difference Measurement Test 2

The same test as in test 3 was performed by using various samples in order to demonstrate that power generation can be performed by using the two-tank configuration, and various types of oxidation-reduction active species can be used, as with the power generating device 100 according to the present embodiment.

### Test Sample

Aqueous solutions as shown in Tables 3 and 4 below were prepared as test samples, and subjected to a test in the same manner as in test 3.

**[Table 3]**

| Sample name | AQDS half reduced (mmol/L) | NA (mmol/L) | NA (half) (mmol/L) | MB (mmol/L) | Nps Blank (mmol/L) |
|---|---|---|---|---|---|
| Oxidation-reduction active species | 1.0 | 1.0 | 1.0 ^{(*1)} | 1.0 | 0 |
| DITH | 0.5 | 0.0 | 0.0 | 0.0 | 0 |
| Nps (COO⁻) | 4.0 | 4.0 | 4.0 | 4.0 | 4.0 |
| NaOH | 2.0 | 2.0 | 2.0 | 2.0 | 2.0 |
| KCl | 30.0 | 30.0 | 30.0 | 30.0 | 30.0 |

| | | | | | |
|---|---|---|---|---|---|
| *1: The sample "NA (half)" was obtained by mixing oxidized nicotinamide and reduced nicotinamide at a molar ratio of 1:1, and the total amount was 1.0 mmol/L. | | | | | |

**[Table 4]**

| Sample name | TMPD (mmol/L) | PHH (mmol/L) | Riboflavin (mmol/L) | Nps Blank (mmol/L) |
|---|---|---|---|---|
| Oxidation-reduction active species | 1.0 | 1.0 | 1.0 | 0 |
| DITH | 0.0 | 0.0 | 0.0 | 0.0 |
| Nps (COO⁻) | 4.0 | 4.0 | 4.0 | 4.0 |
| NaOH | 2.0 | 2.0 | 2.0 | 2.0 |
| KCl | 30.0 | 30.0 | 30.0 | 30.0 |

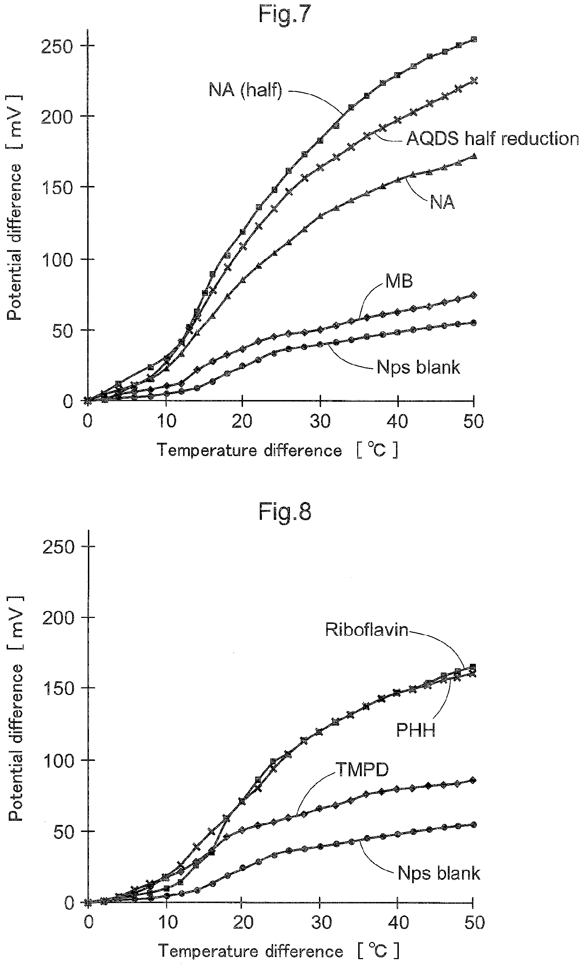

The results of the samples shown in Table 3 are plotted in the graph of FIG. 7. The results of the samples shown in Table 4 are plotted in the graph of FIG. 8. All of the samples exhibited a potential difference larger than the sample "Nps Blank" that did not contain an oxidation-reduction active species. The sample "NA (half)" (a mixed solution of oxidized nicotinamide and reduced nicotinamide (at a molar ratio of 1:1) and four equivalents of Nps) exhibited a potential difference of 254 mV, which was the highest potential difference at a temperature difference of 50°C. The sample "NA (half)" that contained an oxidation-reduction active species exhibited a potential difference about 4.6 times higher than that of the sample "Nps Blank" (54.95 mV).

It was confirmed, from the test described above, that a larger potential difference can be generated when the temperature responsive electrolyte Nps is used together with AQDS (sulfated anthraquinone), NA (nicotinamide), MB (methylene blue), TMPD (N,N,N',N'-tetramethyl-p-phenylenediamine), and PHH (proflavine hemisulfate hydrate) or riboflavin than when the temperature responsive electrolyte is used alone for power generation (the sample "Nps Blank").

### Test 5: Two-Tank Potential Difference Measurement Test 3

The same test as in test 3 was performed by using various samples in order to demonstrate that power generation can be performed by using the two-tank configuration and various types of oxidation-reduction active species can be used, as with the power generating device 100 according to the present embodiment.

### Test Sample

Aqueous solutions as shown in Table 5 below were prepared as test samples, and subjected to a test in the same manner as in test 3.

**[Table 5]**

| | Component | Concentration |
|---|---|---|
| Example 2 | Hydroquinone | 1.0 mmol/L |
| | Temperature responsive electrolyte | 4.0 mmol/L (COO⁻ amount) |
| | Sodium hydroxide | 2.0 mmol/L |
| | Potassium chloride | 30.0 mmol/L |
| Example 3 | Methyl hydroquinone | 1.0 mmol/L |
| | Temperature responsive electrolyte | 4.0 mmol/L (COO⁻ amount) |
| | Sodium hydroxide | 2.0 mmol/L |
| | Potassium chloride | 30.0 mmol/L |
| Comparative Example 4 | Temperature responsive electrolyte | 4.0 mmol/L (COO⁻ amount) |
| | Sodium hydroxide | 2.0 mmol/L |
| | Potassium chloride | 30.0 mmol/L |

As the temperature responsive electrolyte, the same particles as those used in the power generation principle test described above were used.

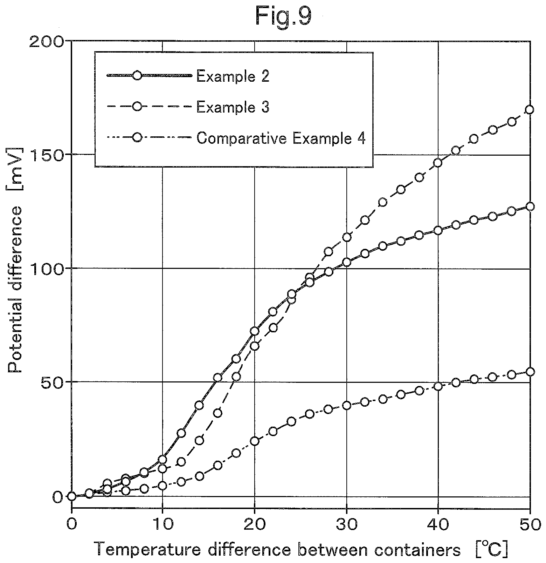

The results of the two-tank potential difference measurement test are plotted in the graph of FIG. 9. In all of Example 2, Example 3, and Comparative Example 4, the potential difference increased with an increase in temperature difference between containers. However, the increase in potential difference in Examples 2 and 3 was much larger than that of Comparative Example 4. It was confirmed, from the test described above, that a larger potential difference can be generated when the temperature responsive electrolyte and the oxidation-reduction reactive species (hydroquinone derivative) are used together (Examples 2 and 3) than when the temperature responsive electrolyte is used alone for power generation (Comparative Example 4).

### Other Embodiments

(1) In the embodiment given above, an example has been described in which a temperature responsive electrolyte that causes a pH change according to temperature and an oxidation-reduction active species are contained in the electrolytic solution. The electrolytic solution and the power generating device may be configured by using, instead of the temperature responsive electrolyte, a substance that causes a pH change in response to other stimuli such as, for example, light.
   The substance that causes a pH change in response to light may be, for example, a composite solution of a methacrylic acid copolymer and an azo dye, or the like, which is reported in the article titled "Photo-Induced Reversible pH Change in Poly(carboxylic acid)-Azo Dye Complex System" (Japanese Journal of Polymer Science and Technology, Vol. 37, No. 4, pp. 293 to 298 (Apr., 1980)). Also, the article titled "A photoinduced pH jump applied to drug release from cucurbit [7] uril" (Chem. Commun., 2011, 47, 8793 to 8795) shows host-guest molecules that undergo a pH change in response to light irradiation. It is also reported that a pH change also occurs when a spiropyran derivative and polymer particles containing a spiropyran derivative are used (J. Am. Chem. Soc., 2011, 133 (37), pp. 14699 to 14703). The power generating device can be configured by using an aqueous solution that contains such a light responsive substance and an oxidation-reduction active species.
(2) In the embodiment given above, a configuration has been described in which heat exchangers are used as specific examples of the cooling mechanism 5 and the heating mechanism 6, and the electrolytic solution S or the like is heated and cooled by exchanging heat between the electrolytic solution S or the like and seawater or hot water. As the cooling mechanism 5 and the heating mechanism 6, any configuration can be used as long as the electrolytic solution S or the like contained in the positive electrode tank 1 and the electrolytic solution S or the like contained in the negative electrode tank 2 can be heated and cooled. It is also possible to use a configuration in which the wall surfaces of the positive electrode tank 1 and the negative electrode tank 2 are heated and cooled, or a configuration in which the electrodes (the positive electrode 3 and the negative electrode 4) are heated and cooled so as to heat and cool the electrolytic solution S or the like.

The configurations disclosed in the embodiments given above (including other embodiments, the same applies hereinafter) may be combined and used with the configurations disclosed in other embodiments as long as there is no contradiction. Also, the embodiments disclosed in the specification of the present application are merely examples. Accordingly, the embodiments of the present invention are not limited thereto.

### Description of Reference Signs

100: Power generating device
1: Positive electrode tank
2: Negative electrode tank
3: Positive electrode
4: Negative electrode
5: Cooling mechanism
6: Heating mechanism
7: Circulation mechanism
7a: First flow path
7b: First pump
7c: Second flow path
7d: Second pump
8: Heat exchange mechanism
11: Aqueous solution tank
12: Cooling water circulation tank
13: High-temperature water circulation tank
14: Low-temperature side electrode
15: High-temperature side electrode
16: Low-temperature heat source
17: High-temperature heat source
18: Current/voltage meter
19: Baffle plate
20: Heat transfer plate
21: Aqueous solution tank
22: Low-temperature side water tank
23: High-temperature side water tank
24: Low-temperature side electrode
25: High-temperature side electrode
26: Low-temperature heat source
27: High-temperature heat source
28: Current meter
31: Low-temperature side tank
32: High-temperature side tank
33: Low-temperature bath
34: High-temperature bath
35: Low-temperature side electrode
36: High-temperature side electrode
37: Salt bridge
38: Voltage meter
S: Electrolytic solution (electrolytic aqueous solution)

## Claims

1. An electrolytic solution comprising:
a temperature responsive electrolyte that is an electrolyte whose pKa varies according to temperature; and
an oxidation-reduction active species (excluding a hydroquinone derivative that is a monocyclic aromatic compound that has a hydroquinone skeleton),
wherein the temperature responsive electrolyte is a molecule that has a polar group, a hydrophobic group, and an ionizable functional group.

2. An electrolytic solution according to claim 1, wherein
the oxidation-reduction active species is a member selected from N,N,N',N'-tetramethyl-p-phenylenediamine or a derivative thereof, nicotinamide or a derivative thereof, a proflavine hemisulfate hydrate or a derivative thereof, riboflavin or a derivative thereof, sulfated anthraquinone or a derivative thereof, naphthoquinone or a derivative thereof, and methylene blue or a derivative thereof.

3. A power generating device that performs power generation by using the electrolytic solution according to any one of claims 1 to 2, the power generating device comprising:
a positive electrode;
a negative electrode;
a heating mechanism; and
a cooling mechanism,
wherein the positive electrode and the negative electrode are immersed in the electrolytic solution,
the heating mechanism heats the electrolytic solution that is present in the vicinity of one of the positive electrode and the negative electrode, and
the cooling mechanism cools the electrolytic solution that is present in the vicinity of the other one of the positive electrode and the negative electrode.

4. The power generating device according to claim 3,
wherein the heating mechanism heats the electrolytic solution to a temperature higher than a phase transition temperature of the temperature responsive electrolyte, and
the cooling mechanism cools the electrolytic solution to a temperature lower than the phase transition temperature of the temperature responsive electrolyte.

5. The power generating device according to claim 3 or 4, comprising:
a positive electrode tank;
a negative electrode tank; and
a circulation mechanism,
wherein the electrolytic solution is contained in the positive electrode tank and the negative electrode tank,
the positive electrode is in contact with the electrolytic solution contained in the positive electrode tank,
the negative electrode is in contact with the electrolytic solution contained in the negative electrode tank,
the heating mechanism heats either one of the electrolytic solution contained in the positive electrode tank and the electrolytic solution contained in the negative electrode tank,
the cooling mechanism cools the other one of the electrolytic solution contained in the positive electrode tank and the electrolytic solution contained in the negative electrode tank, and
the circulation mechanism circulates the electrolytic solution between the positive electrode tank and the negative electrode tank.

6. The power generating device according to claim 5, comprising:
a heat exchange mechanism,
wherein the heat exchange mechanism performs heat exchange between the electrolytic solution delivered to the positive electrode tank by the circulation mechanism and the electrolytic solution delivered to the negative electrode tank by the circulation mechanism.

7. An electrolytic aqueous solution comprising:
a temperature responsive electrolyte that is an electrolyte whose pKa varies according to temperature; and
an oxidation-reduction reactive species that is a hydroquinone derivative that is a monocyclic aromatic compound that has a hydroquinone skeleton,
wherein the temperature responsive electrolyte is a molecule that has a polar group, a hydrophobic group, and an ionizable functional group.

8. An electrolytic aqueous solution according to claim 7,
wherein the hydroquinone derivative does not precipitate in the electrolytic aqueous solution.

9. The electrolytic aqueous solution according to any one of claims 7 to 8,
wherein the oxidation-reduction reactive species is one selected from hydroquinone and methyl hydroquinone.

10. A power generating device that performs power generation by using the electrolytic aqueous solution according to any one of claims 7 to 9, the power generating device comprising:
a positive electrode;
a negative electrode;
a heating mechanism; and
a cooling mechanism,
wherein the positive electrode and the negative electrode are immersed in the electrolytic aqueous solution,
the heating mechanism heats the electrolytic aqueous solution that is present in the vicinity of one of the positive electrode and the negative electrode, and
the cooling mechanism cools the electrolytic aqueous solution that is present in the vicinity of the other one of the positive electrode and the negative electrode.

11. The power generating device according to claim 10,
wherein the heating mechanism heats the electrolytic aqueous solution to a temperature higher than a phase transition temperature of the temperature responsive electrolyte, and
the cooling mechanism cools the electrolytic aqueous solution to a temperature lower than the phase transition temperature of the temperature responsive electrolyte.

12. The power generating device according to claim 10 or 11, comprising:
a positive electrode tank;
a negative electrode tank; and
a circulation mechanism,
wherein the electrolytic aqueous solution is contained in the positive electrode tank and the negative electrode tank,
the positive electrode is in contact with the electrolytic aqueous solution contained in the positive electrode tank,
the negative electrode is in contact with the electrolytic aqueous solution contained in the negative electrode tank,
the heating mechanism heats one of the electrolytic aqueous solution contained in the positive electrode tank and the electrolytic aqueous solution contained in the negative electrode tank,
the cooling mechanism cools the other one of the electrolytic aqueous solution contained in the positive electrode tank and the electrolytic aqueous solution contained in the negative electrode tank, and
the circulation mechanism circulates the electrolytic aqueous solution between the positive electrode tank and the negative electrode tank.

13. The power generating device according to claim 12, comprising:
a heat exchange mechanism,
wherein the heat exchange mechanism performs heat exchange between the electrolytic aqueous solution delivered to the positive electrode tank by the circulation mechanism and the electrolytic aqueous solution delivered to the negative electrode tank by the circulation mechanism.

## Patentansprüche

1. Elektrolytlösung, umfassend:
einen thermoresponsiven Elektrolyten, der ein Elektrolyt ist, dessen pKa-Wert mit der Temperatur variiert; und
eine oxidations-reduktions-aktive Spezies (die ein Hydrochinon-Derivat ausschließt, das eine monozyklische aromatische Verbindung ist, die ein Hydrochinon-Skelett hat),
wobei der thermoresponsive Elektrolyt ein Molekül ist, das eine polare Gruppe, eine hydrophobe Gruppe und eine ionisierbare Funktionsgruppe hat.

2. Elektrolytlösung gemäß Anspruch 1, wobei
die oxidations-reduktions-aktive Spezies ein Mitglied ist, das aus N,N,N',N'-Tetramethyl-p-phenylendiamin oder einem Derivat davon, Nicotinamid oder einem Derivat davon, einem Proflavin-Hemisulfat-Hydrat oder einem Derivat davon, Riboflavin oder einem Derivat davon, sulfatiertem Anthrachinon oder einem Derivat davon, Naphthochinon oder einem Derivat davon und Methylenblau oder einem Derivat davon ausgewählt ist.

3. Stromerzeugungsvorrichtung, die eine Stromerzeugung unter der Verwendung der Elektrolytlösung gemäß einem der Ansprüche 1 bis 2 durchführt, wobei die Stromerzeugungsvorrichtung umfasst:
eine positive Elektrode;
eine negative Elektrode;
einen Heizmechanismus; und
einen Kühlmechanismus,
wobei die positive Elektrode und die negative Elektrode in die Elektrolytlösung getaucht sind,
der Heizmechanismus die Elektrolytlösung aufheizt, die in der Nachbarschaft einer aus der positiven Elektrode und der negativen Elektrode vorhanden ist, und
der Kühlmechanismus die Elektrolytlösung abkühlt, die in der Nachbarschaft der anderen aus der positiven Elektrode und der negativen Elektrode vorhanden ist.

4. Stromerzeugungsvorrichtung gemäß Anspruch 3,
wobei der Heizmechanismus die Elektrolytlösung auf eine Temperatur aufheizt, die höher als eine Phasenübergangstemperatur des thermoresponsiven Elektrolyten ist, und
der Kühlmechanismus die Elektrolytlösung auf eine Temperatur abkühlt, die niedriger als die Phasenübergangstemperatur des thermoresponsiven Elektrolyten ist.

5. Stromerzeugungsvorrichtung gemäß Anspruch 3 oder 4, umfassend:
einen Tank für die positive Elektrode;
einen Tank für die negative Elektrode; und
einen Zirkulationsmechanismus,
wobei die Elektrolytlösung in dem Tank für die positive Elektrode und in dem Tank für die negative Elektrode enthalten ist,
wobei die positive Elektrode mit der in dem Tank für die positive Elektrode enthaltenen Elektrolytlösung in Kontakt ist,
die negative Elektrode mit der in dem Tank für die negative Elektrode enthaltenen Elektrolytlösung in Kontakt ist,
der Heizmechanismus eine aus der in dem Tank für die positive Elektrode enthaltenen Elektrolytlösung und der in dem Tank für die negative Elektrode enthaltenen Elektrolytlösung aufheizt,
der Kühlmechanismus die andere aus der in dem Tank für die positive Elektrode enthaltenen Elektrolytlösung und der in dem Tank für die negative Elektrode enthaltenen Elektrolytlösung abkühlt, und
der Zirkulationsmechanismus die Elektrolytlösung zwischen dem Tank für die positive Elektrode und dem Tank für die negative Elektrode zirkulieren lässt.

6. Stromerzeugungsvorrichtung gemäß Anspruch 5, umfassend:
einen Wärmetauschmechanismus,
wobei der Wärmetauschmechanismus einen Wärmetausch zwischen der durch den Zirkulationsmechanismus an den Tank für die positive Elektrode gelieferten Elektrolytlösung und der durch den Zirkulationsmechanismus an den Tank für die negative Elektrode gelieferten Elektrolytlösung durchführt.

7. Wässrige Elektrolytlösung, umfassend:
einen thermoresponsiven Elektrolyten, der ein Elektrolyt ist, dessen pKa-Wert mit der Temperatur variiert; und
eine oxidations-reduktions-reaktive Spezies, die ein Hydrochinon-Derivat ist, das eine monozyklische aromatische Verbindung ist, die ein Hydrochinon-Skelett hat,
wobei der thermoresponsive Elektrolyt ein Molekül ist, das eine polare Gruppe, eine hydrophobe Gruppe und eine ionisierbare Funktionsgruppe hat.

8. Wässrige Elektrolytlösung gemäß Anspruch 7,
wobei das Hydrochinon-Derivat in der wässrigen Elektrolytlösung nicht ausfällt.

9. Wässrige Elektrolytlösung gemäß einem der Ansprüche 7 bis 8,
wobei die oxidations-reduktions-reaktive Spezies eine ist, die aus Hydrochinon und Methylhydrochinon ausgewählt ist.

10. Stromerzeugungsvorrichtung, die eine Stromerzeugung unter der Verwendung der wässrigen Elektrolytlösung gemäß einem der Ansprüche 7 bis 9 durchführt, wobei die Stromerzeugungsvorrichtung umfasst:
eine positive Elektrode;
eine negative Elektrode;
einen Heizmechanismus; und
einen Kühlmechanismus,
wobei die positive Elektrode und die negative Elektrode in die wässrige Elektrolytlösung getaucht sind,
der Heizmechanismus die wässrige Elektrolytlösung aufheizt, die in der Nachbarschaft einer aus der positiven Elektrode und der negativen Elektrode vorhanden ist, und
der Kühlmechanismus die wässrige Elektrolytlösung abkühlt, die in der Nachbarschaft der anderen aus der positiven Elektrode und der negativen Elektrode vorhanden ist.

11. Stromerzeugungsvorrichtung gemäß Anspruch 10,
wobei der Heizmechanismus die wässrige Elektrolytlösung auf eine Temperatur aufheizt, die höher als eine Phasenübergangstemperatur des thermoresponsiven Elektrolyten ist, und
der Kühlmechanismus die wässrige Elektrolytlösung auf eine Temperatur abkühlt, die niedriger als die Phasenübergangstemperatur des thermoresponsiven Elektrolyten ist.

12. Stromerzeugungsvorrichtung gemäß Anspruch 10 oder 11, umfassend:
einen Tank für die positive Elektrode;
einen Tank für die negative Elektrode; und
einen Zirkulationsmechanismus,
wobei die wässrige Elektrolytlösung in dem Tank für die positive Elektrode und in dem Tank für die negative Elektrode enthalten ist,
wobei die positive Elektrode mit der in dem Tank für die positive Elektrode enthaltenen wässrigen Elektrolytlösung in Kontakt ist,
die negative Elektrode mit der in dem Tank für die negative Elektrode enthaltenen wässrigen Elektrolytlösung in Kontakt ist,
der Heizmechanismus eine aus der in dem Tank für die positive Elektrode enthaltenen wässrigen Elektrolytlösung und der in dem Tank für die negative Elektrode enthaltenen wässrigen Elektrolytlösung aufheizt,
der Kühlmechanismus die andere aus der in dem Tank für die positive Elektrode enthaltenen wässrigen Elektrolytlösung und der in dem Tank für die negative Elektrode enthaltenen wässrigen Elektrolytlösung abkühlt, und
der Zirkulationsmechanismus die wässrige Elektrolytlösung zwischen dem Tank für die positive Elektrode und dem Tank für die negative Elektrode zirkulieren lässt.

13. Stromerzeugungsvorrichtung gemäß Anspruch 12, umfassend:
einen Wärmetauschmechanismus,
wobei der Wärmetauschmechanismus einen Wärmetausch zwischen der durch den Zirkulationsmechanismus an den Tank für die positive Elektrode gelieferten wässrigen Elektrolytlösung und der durch den Zirkulationsmechanismus an den Tank für die negative Elektrode gelieferten wässrigen Elektrolytlösung durchführt.

## Revendications

1. Solution électrolytique comprenant :
un électrolyte sensible à la température qui est un électrolyte dont le pKa varie en fonction de la température ; et
une espèce active d'oxydoréduction (à l'exclusion d'un dérivé d'hydroquinone qui est un composé aromatique monocyclique qui a un squelette d'hydroquinone),
dans lequel l'électrolyte sensible à la température est une molécule qui a un groupe polaire, un groupe hydrophobe , et un groupe fonctionnel ionisable.

2. Solution électrolytique selon la revendication 1, dans laquelle
l'espèce active d'oxydoréduction est un élément sélectionné parmi de la N,N,N',N'-tétraméthyl-p-phénylènediamine ou un dérivé de celle-ci, nicotinamide ou un dérivé de celui-ci, un hydrate de proflavine hémisulfate ou un dérivé de celui-ci, riboflavine ou un dérivé de celle-ci, anthraquinone sulfatée ou un dérivé de celle-ci, naphtoquinone ou un dérivé de celle-ci, et bleu de méthylène ou un dérivé de celui-ci.

3. Dispositif générateur d'énergie qui effectue une génération d'énergie en utilisant la solution électrolytique selon l'une des revendications 1 à 2, le dispositif générateur d'énergie comprenant :
une électrode positive ;
une électrode négative ;
un mécanisme de chauffage ; et
un mécanisme de refroidissement,
dans lequel l'électrode positive et l'électrode négative sont immergées dans la solution électrolytique,
le mécanisme de chauffage chauffe la solution électrolytique qui est présente à proximité de l'une de l'électrode positive et de l'électrode négative, et le mécanisme de refroidissement refroidit la solution électrolytique qui est présente à proximité de l'autre de l'électrode positive et de l'électrode négative.

4. Dispositif générateur d'énergie selon la revendication 3,
dans lequel le mécanisme de chauffage chauffe la solution électrolytique à une température supérieure à une température de transition de phase de l'électrolyte sensible à la température, et
le mécanisme de refroidissement refroidit la solution électrolytique à une température inférieure à la température de transition de phase de l'électrolyte sensible à la température.

5. Dispositif générateur d'énergie selon la revendication 3 ou 4, comprenant :
un réservoir à électrode positive ;
un réservoir à électrode négative ; et
un mécanisme de circulation,
dans lequel la solution électrolytique est contenue dans le réservoir à électrode positive et le réservoir à électrode négative,
l'électrode positive est en contact avec la solution électrolytique contenue dans le réservoir à électrode positive,
l'électrode négative est en contact avec la solution électrolytique contenue dans le réservoir à électrode négative,
le mécanisme de chauffage chauffe l'une de la solution électrolytique contenue dans le réservoir à électrode positive et de la solution électrolytique contenue dans le réservoir à électrode négative,
le mécanisme de refroidissement refroidit l'autre de la solution électrolytique contenue dans le réservoir à électrode positive et de la solution électrolytique contenue dans le réservoir à électrode négative, et
le mécanisme de circulation fait circuler la solution électrolytique entre le réservoir à électrode positive et le réservoir à électrode négative.

6. Dispositif générateur d'énergie selon la revendication 5, comprenant :
un mécanisme d'échange de chaleur,
dans lequel le mécanisme d'échange de chaleur effectue un échange de chaleur entre la solution électrolytique délivrée dans le réservoir à électrode positive par le mécanisme de circulation et la solution électrolytique délivrée dans le réservoir à électrode négative par le mécanisme de circulation.

7. Solution aqueuse électrolytique comprenant :
un électrolyte sensible à la température qui est un électrolyte dont le pKa varie en fonction de la température ; et
une espèce réactive d'oxydoréduction qui est un dérivé d'hydroquinone qui est un composé aromatique monocyclique qui a un squelette d'hydroquinone,
dans laquelle l'électrolyte sensible à la température est une molécule qui a un groupe polaire, un groupe hydrophobe, et un groupe fonctionnel ionisable.

8. Solution aqueuse électrolytique selon la revendication 7,
dans laquelle le dérivé hydroquinone ne précipite pas dans la solution aqueuse électrolytique.

9. Solution aqueuse électrolytique selon l'une des revendications 7 à 8,
dans laquelle l'espèce réactive d'oxydoréduction est une espèce sélectionnée parmi de l'hydroquinone et de la méthylhydroquinone.

10. Dispositif générateur d'énergie qui effectue une génération d'énergie en utilisant la solution aqueuse éledrolytique selon l'une des revendications 7 à 9, le dispositif générateur d'énergie comprenant :
une électrode positive ;
une électrode négative ;
un mécanisme de chauffage ; et
un mécanisme de refroidissement,
dans lequel l'électrode positive et l'électrode négative sont immergées dans la solution aqueuse électrolytique,
le mécanisme de chauffage chauffe la solution aqueuse électrolytique qui est présente à proximité de l'une de l'électrode positive et de l'électrode négative, et
le mécanisme de refroidissement refroidit la solution aqueuse électrolytique qui est présente à proximité de l'autre de l'électrode positive et de l'électrode négative.

11. Dispositif générateur d'énergie selon la revendication 10,
dans lequel le mécanisme de chauffage chauffe la solution aqueuse électrolytique à une température supérieure à une température de transition de phase de l'électrolyte sensible à la température, et
le mécanisme de refroidissement refroidit la solution aqueuse électrolytique à une température inférieure à la température de transition de phase de l'électrolyte sensible à la température.

12. Dispositif générateur d'énergie selon la revendication 10 ou 11, comprenant :
un réservoir à électrode positive ;
un réservoir à électrode négative ; et
un mécanisme de circulation,
dans lequel la solution aqueuse électrolytique est contenue dans le réservoir à électrode positive et le réservoir à électrode négative,
l'électrode positive est en contact avec la solution aqueuse électrolytique contenue dans le réservoir à électrode positive,
l'électrode négative est en contact avec la solution aqueuse électrolytique contenue dans le réservoir à électrode négative,
le mécanisme de chauffage chauffe l'une de la solution aqueuse électrolytique contenue dans le réservoir à électrode positive et de la solution aqueuse électrolytique contenue dans le réservoir à électrode négative,
le mécanisme de refroidissement refroidit l'autre de la solution aqueuse électrolytique contenue dans le réservoir à électrode positive et de la solution aqueuse électrolytique contenue dans le réservoir à électrode négative, et
le mécanisme de circulation fait circuler la solution aqueuse électrolytique entre le réservoir à électrode positive et le réservoir à électrode négative.

13. Dispositif générateur d'énergie selon la revendication 12, comprenant :
un mécanisme d'échange de chaleur,
dans lequel le mécanisme d'échange de chaleur effectue un échange de chaleur entre la solution aqueuse électrolytique délivrée dans le réservoir à électrode positive par le mécanisme de circulation et la solution aqueuse électrolytique délivrée dans le réservoir à électrode négative par le mécanisme de circulation.
